# EUROPEAN PATENT SPECIFICATION

(11) **EP 2 618 313 B1**
(45) Date of publication and mention of the grant of the patent: **30.07.2014**
(21) Application number: 12197154.3
(22) Date of filing: 14.12.2012
(51) Int. Cl.: G06T 19/00, A61B 5/00, G06F 19/00

(54) **Process to generate a computeraccessible medium comprising information on the functioning of a joint**
Verfahren zum Erzeugen eines computerzugänglichen Mediums, umfassend Informationen über die Funktionsweise eines Verbindungsteils
Procédé pour générer un support accessible par informatique comprenant des informations sur le fonctionnement d'une articulation

(30) Priority: 19.01.2012 NL 2008143; 09.03.2012 NL 2008437
(43) Date of publication of application: 24.07.2013
(73) Proprietor: Clinical Graphics B.V., 2629 JD Delft (NL)
(72) Inventor: Krekel, Peter Roelof, 2593 TB Den Haag (NL)
(74) Representative: Cramwinckel, Michiel

(56) References cited:
- EP-A2- 1 059 614
- US-A1- 2010 198 067
- KREKEL ET AL: "Interactive simulation and comparative visualisation of the bone-determined range of motion of the human shoulder", SIMULATION UND VISUALISIERUNG 2006 (SIMVIS 2006), SCS-VERLAG MAGDEBURG, GERMANY, 1 January 2006 (2006-01-01), pages 275-287, XP009163497, ISBN: 978-3-936150-46-9
- BEI Y ET AL: "Multibody dynamic simulation of knee contact mechanics", MEDICAL ENGINEERING & PHYSICS, BUTTERWORTH-HEINEMANN, GB, vol. 26, no. 9, 1 November 2004 (2004-11-01), pages 777-789, XP004654047, ISSN: 1350-4533, DOI: 10.1016/J.MEDENGPHY.2004.07.004

## Description

The invention relates to a process to generate a computer-accessible medium comprising information on the functioning of a joint.

From Krekel2006 (Krekel PR, Botha CP, Valstar ER, DeBruin PW, Post FH, Rozing PM. Interactive simulation and comparative visualisation of the bone-determined range of motion of the human shoulder. In: Schulze T, Horton G, Preim B, Schlechtweg S Proc of SimVis. SCS Publishing House Erlangen 2006; 275-288.) it is known that surgeons can plan operations on the joint using 3 dimensional models of the specific joint. The 3 dimensional model is developed using CT-data of the joint and subsequently extracting surface models. Bone-determined Range of motion (ROM) is automatically determined by systematically reorienting, for example, the humerus with placed humeral component in all directions, starting from an initial abduction of 45, while checking for collisions with a collision detection algorithm. ROM is the area through which the humerus may be freely and painlessly moved.

A disadvantage of the above method is that a surgeon needs knowledge of the functioning of the computer program to run the 3 dimensional model. Furthermore calculation power is required to run this 3-dimensional model requiring more complicated and expensive hardware at the end user. A next disadvantage is that the surgeon or the radiologist will need to spend time to operate the computer program and to assess if the ROM of a joint is limited.

The present invention aims at simplifying the use of 3-dimensional (3D) models when analysing the ROM of a specific joint. This is achieved by the following process. Process to generate a computer-accessible medium comprising information on the functioning of a joint, wherein the computer-accessible medium comprises an executable instruction to run a multimedia object by
(i) obtaining an image dataset with the aid of a radiological examination of the joint,
(ii) building a 3D-computer model of the joint ,
(iii) performing a biomechanical or kinematic simulation using the computer model,
(iv) receiving the biomechanical or kinematic simulation results as a multimedia object and integrating the multimedia object with a computer-accessible medium.

The above process is advantageous because the end user, for example the radiologist or the surgeon, can easily access the computer-accessible medium and does not have to install any additional software to run the integrated multimedia object. The only software to install is the software to run the computer-accessible medium. Preferably the computer-accessible medium will require software which is generally available or installed on computers of the end users. The computer required to run the computer accessible medium is typically simpler than the computer to perform the biomechanical or kinematic simulation of the joint in step (iii). Thus with the present process it has become possible to review the biomechanical and/or kinematic functioning of a joint using a simple software program without having to use the heavy computing power to run a biomechanical or kinematic simulation of the joint. Other advantages of the present invention will be discussed when describing the invention in detail.

In step (i) the image dataset is suitably obtained by a computed tomography (CT) system, a magnetic resonance imaging (MRI) system, a positron emission tomography system, an x-ray device or an ultrasound device. Preferably the image dataset is obtained by a computed tomography system or by a magnetic resonance imaging (MRI) system.

In step (ii) a model is build of the joint using the image dataset obtained in step (i). Building a model may be performed as described in Krekel2010 (Krekel PR, Valstar ER, Post FH, Rozing PM, Botha CP. Combined surface and volume processing for fused joint segmentation. The International Journal for Computer Assisted Radiology and Surgery 2010; 5(3), 263-273.). Or as for example as described in US2011/0235887. The model is a computer model and will be further referred to as a 3D-computer model. The 3D-computer model enables the user to visual ise movement of a joint.

In step (iii) a biomechanical or kinematic simulation of the joint is performed using the 3D-computer model. Preferably more than one movement of the joint is simulated. In these simulations it may be found that bone collision occurs at a specific movement of the joint. Suitably a dataset based on the simulation results is generated in step (iii), comprising data relating to the position and orientation of the joint members and data relating to the points of bone collision if found in the simulation. Preferably the available joint space between the joint members is part of the dataset. Preferably a number of movements of the joint are performed to cover the whole range of motion (ROM) of the joint. In this manner a complete dataset is obtained for the ROM of the joint under investigation. This dataset may also comprise information regarding the patient such as name, age, sex, medical history and patient identification number. Preferably the dataset also comprises data relating to proposed surgical modifications. The dataset so obtained is suitably operable with the 3D-model of the joint as obtained in step (ii) to produce a time ordered series of frames, wherein each frame consists of a 2D or 3D representation of a position and an orientation and/or a state of tissue types in the joint.

Preferably the dataset and the 3D-computer model is combined with an interactive viewer in step (iv) to obtain an interactive 3D-model as the multimedia object which is integrated with the computer-accessible medium. A preferred interactive viewer is a programmable viewer based on JavaScript.

In step (iii) the 3D-model is preferably used to obtain a relevant biomechanical or kinematic simulation result or results and in step (iv) the relevant biomechanical or kinematic simulation results are received as a multimedia object and integrated with a computer-accessible medium. In the context of the present invention a 'relevant' result either relates to the malfunctioning of a joint or in the absence of any malfunctioning of the joint to a result showing a healthy functioning of the joint. Because the multimedia object is created from the relevant results of the biomechanical or kinematic simulation it is possible to give the end user direct and immediate access to these relevant results. This simplifies the speed of diagnostic evaluation and reduces the risk of missing relevant ROM deterioration.

Suitably the relevant malfunctioning of the joint relate to the function of deformed joints, of injured joints or joints with planned or already present prosthetic parts. Suitably the 3D-computer model of the joint is used in step (iii) to analyse a range of motion (ROM) of the joint , determine situations of bone collisions or situations of no bone collision as the relevant biomechanical or kinematic simulation results. In this process a motion of the joint is analysed. Preferably a number of predetermined motion patterns are tested. These motion patterns are typical for a specific joint. If no bone collision is determined a next motion of the joint is analysed. If a bone collision is determined a multimedia object is created showing the movement of the joint and the bone collision as the relevant biomechanical or kinematic simulation result. Preferably the area of bone collision is indicated by a contrasting colour, for example red, relative to the colour used for the bone parts, for example grey, of the joint. The above analysis of the motion of the joint within the range of movements (ROM) is preferably repeated until all possible movements have been analysed. If more than one bone collision is determined more multimedia objects may be created showing the relevant biomechanical or kinematic simulation results. If no bone collision is determined within the ROM it is preferred that a multimedia object is created showing one or multiple healthy motion patterns of the joint as the relevant result. The relevant biomechanical or kinematic simulation results are suitably saved as a multimedia object and more preferably as part of a dataset as described above. The dataset may in combination with the 3D-model be viewed as a time ordered series of frames, each frame consisting of a 2D or 3D representation of a position and an orientation and/or a state of tissue types in the joint. The multimedia object or dataset are used in step (iv).

Preferably the computer-accessible medium of step (iv) is a Portable Document Format, also called PDF document or a HTML document. The PDF document can be read by the free Adobe® Acrobat Reader(R) viewer starting from the Version 7.0. Adobe® Acrobat Reader is a product obtainable from Adobe Systems. Such a viewer should be able to present 2D or 3D multimedia objects as embedded in the PDF document on the screen of the user. The HTML document can be read with an appropriate Internet browser, such as Microsoft Internet Explorer or Google Chrome. The browser will enable to present the 2D or 3D multimedia objects on the screen of the user.

An appropriate PDF Viewer or Internet Browser is practically present on each computer, so that the end-user of the computer-accessible medium does not have to install any additional software in order to retrieve the relevant biomechanical or kinematic simulation results as a multimedia object. The multimedia object is therefore suitably a device-independent object.

Step (iv) is preferably performed using Acrobat Adobe Pro Extended when integrating the multimedia object with a PDF document. Integrating 2D and 3D multimedia objects into PDF documents is well known feature of this software. Preferably the dataset obtained in step (iii) and the 3D-computer model obtained in step (ii) is combined with an interactive viewer in step (iv) to obtain an interactive 3D-model as the multimedia object which is embedded with the computer-accessible medium. A preferred interactive viewer is a programmable viewer based on JavaScript. The interactive viewer may be programmed to provide the user executable buttons, for example mouse operated executable buttons, which can initiate predefined tasks. An example of predefined task may be a time ordered series of frames, each frame consisting of a 2D or 3D representation of a position and an orientation and/or a state of tissue types in the joint, for the 'relevant' biomechanical or kinematic simulation result. Another example may be the manipulation by the user of the embedded 3D model, wherein the visual presentation of the joint movements is directly shown in the multimedia object. Another example is where a visual presentation is shown of the simulated range of motion for relevant motion patterns of the joint.

The computer-accessible medium, preferably the PDF or HTML document, may also comprise additional information regarding the functioning of the joint. Additional information may for example be information indicating the patient, like for example name, age, sex, medical history and patient identification number. Preferably the computer-accessible medium comprises additional information derived from the image dataset with the aid of a radiological examination of the joint as obtained in step (i) or additional information derived from the model as obtained in step (ii). More preferably computer-accessible medium comprises an interactive 3D model of the joint based on the computer model as obtained in step (ii) and wherein the computer-accessible medium comprises executable instructions to interact with the 3D model. The interactive 3D model of the joint is suitably a device-independent object.

The invention is also directed to the following process. Process to generate a computer-accessible medium comprising information on the functioning of a joint, wherein the computer-accessible medium comprises an executable instruction to run a multimedia object by
(a) obtaining an image dataset with the aid of a radiological examination of the joint,
(b) building a 3D-computer model of the joint,
(c) performing a biomechanical or kinematic simulation of the joint using the computer model wherein more than one movement of the joint is simulated and wherein bone collision may happen at a specific movement of the joint, and creating a dataset based on the resulting simulation results comprising data relating to the oriental positioning of the joint members for a specific movement and data relating to the points of bone collision provided bone collision is detected by the simulation,
(d) combining an interactive viewer, the dataset and the 3D-computer model and embedding the resulting interactive 3D-model in a computer-accessible medium.

Step (a) and (b) may be performed as steps (i) and (ii). Steps (c) and (d) may be performed as described for steps (iii) and (iv). When further reference is made to steps (i), (ii), (iii) and (iv) one may also read steps (a), (b), (c) and (d).Steps (ii)-(iv) are preferably performed on a single computer platform and more preferably in an automated process requiring minimal interaction. The product of this process, namely the computer-accessible medium, can be provided to the end-user, e.g. the surgeon or the radiologist, by email or via a suitable carrier, like for example a USB stick and the like or be placed on a central data carrier, for example a server, which is accessible by said end-user. Steps (ii)-(iv) may be performed at a different location than the location at which step (i) is performed. In a preferred embodiment steps (ii)-(iv) are performed at a single location, wherein step (i) is performed at different locations. In this manner step (i) can be performed near the patient's regular hospital, while the production of the computer-accessible medium products can be performed at a more central location servicing more than one hospital. In this manner heavy computing power and software required to perform steps (ii)-(iv) have to be present at less locations than the number of locations where step (i) is performed.

The invention is also directed to the use of the computer-accessible medium as obtained by the above process to diagnose the functioning of a joint. The end user preferably has a suitable software installed to view the computer-accessible medium. In case the computer-accessible medium is a PDF document a PDF reader such as Acrobat Reader 7.0 or higher is preferably installed on his or hers computer or on the central computer network. In case the computer-accessible medium is a HTML document a suitable Internet Browser is installed on his or hers computer or on the central computer network.

The computer-accessible medium as obtained by the above process may also be used to explain the functioning of a joint to a patient. This may for example be when visiting the surgeon or radiologist or at home.

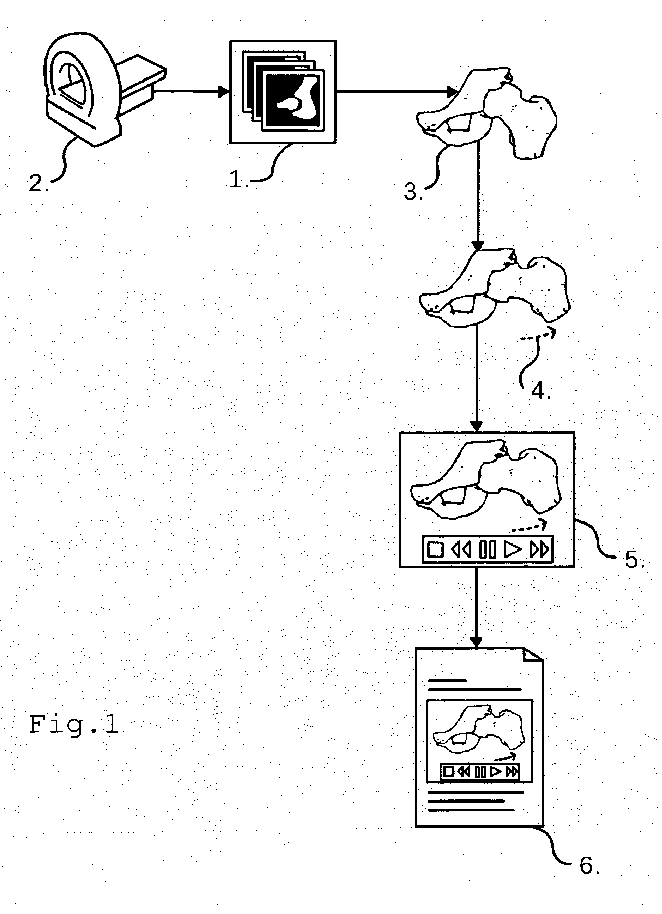

The invention will be illustrated by means of the following Figures. Figure 1 shows the process steps to generate a computer-accessible medium comprising information on the functioning of a joint. In step (i) an image dataset (1) with the aid of a radiological examination of the joint (2). In a step (ii) a computer model of the joint (3) is build. Using this model biomechanical or kinematic simulation of the joint (3) is performed and one or more relevant biomechanical or kinematic simulation results (4) are determined. The relevant biomechanical or kinematic simulation are received in step (iv) as a multimedia object (5). This multimedia object (5) is subsequently integrating with a computer-accessible medium (6).

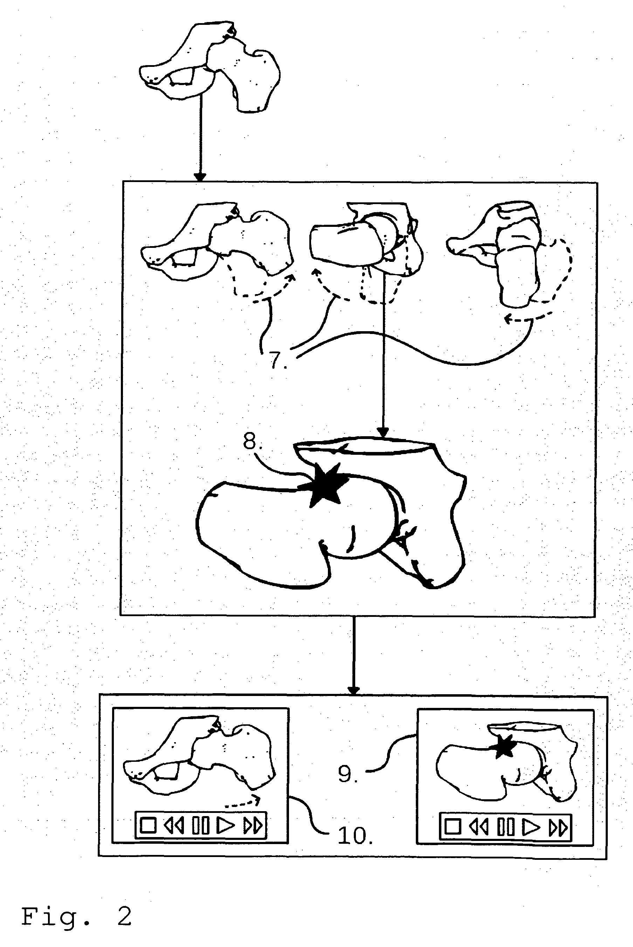

Figure 2 shows a process wherein the computer model of the joint is used to analyse a range of motions of the joint (7). If a situation of bone collisions (8) is determined it is qualified as a relevant biomechanical or kinematic simulation result. A multimedia object (9) is created showing the relevant biomechanical or kinematic simulation result. If no bone collision is determined within the ROM a multimedia object (10) is created showing the functioning of a healthy joint.

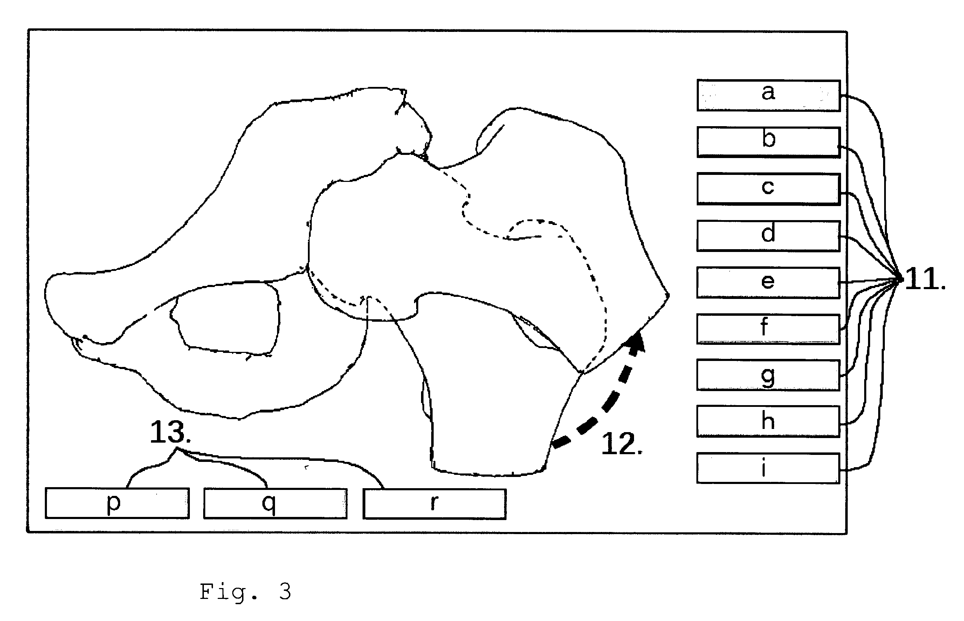

Figure 3 shows an example of how the relevant biomechanical or kinematic simulation result(s) may be presented as an embedded document in a computer-accessible medium. Figure 3 only shows the box with the embedded information. Several executable buttons (11a-i) are present which the user can activate. Button (11-a) is labelled 'abduction' and will show the biomechanically simulated available range of motion for an abduction (sideways) motion pattern as a multimedia object (12). The end-user will know that by executing this labelled button the relevant biomechanical or kinematic simulation result will be presented. If he or she requires more information regarding the joint, like manipulating an embedded 3D model, button (13p, 13q or 13r) may be executed. Manipulations of an embedded 3D model are directly shown in the multimedia object and may lead to different biomechanical simulation results. The executable buttons (11a-i) will now show the biomechanically simulated available range of motion for relevant motion patterns of the manipulated embedded 3D model, rather than the original 3D model.

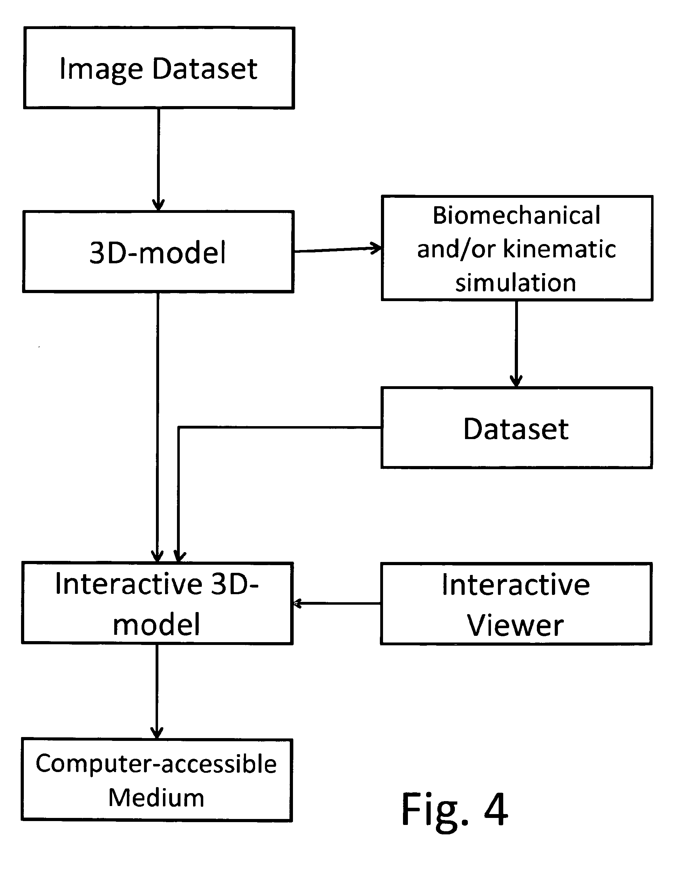

Figure 4 illustrates a preferred embodiment of the present invention wherein in step (iii) a dataset is generated. As shown first an Image Dataset is obtained of the joint. Based on the Image Dataset a 3D-model is build and subsequently used to perform a biomechanical and/or kinematic simulation. This simulation generates data relating to the oriental positioning of the joint members for a specific movement and data relating to the points of bone collision provided bone collision is detected by the simulation. The Dataset is combined with the 3D-model and an Interactive Viewer to obtain an interactive 3D-model. The interactive 3D-model is subsequently embedded in a computer-accessible medium.

## Claims

1. Process to generate a computer-accessible medium comprising information on the functioning of a joint, wherein the computer-accessible medium comprises an executable instruction to run a multimedia object by
(i) obtaining an image dataset with the aid of a radiological examination of the joint,
(ii) building a 3D-computer model of the joint using the image dataset obtained in step (i),
(iii) performing a biomechanical or kinematic simulation using the computer model,
(iv) receiving the biomechanical or kinematic simulation results as a multimedia object and integrating the multimedia object with a computer-accessible medium.

2. Process according to claim 1, wherein in step (iii) one or more relevant biomechanical or kinematic simulation results are determined and that in step (iv) the relevant biomechanical or kinematic simulation results are received as a multimedia object and integrated with a computer-accessible medium.

3. Process according to any one of claims 1-2, wherein the computer-accessible medium comprises an interactive 3D model of the joint based on the computer model as obtained in step (ii) and wherein the computer-accessible medium comprises executable instructions to interact with the 3D model.

4. Process according to claim 3, wherein in step (iii) a biomechanical or kinematic simulation of the joint is performed using the 3D-computer model, wherein more than one movement of the joint is simulated and wherein bone collision may happen at a specific movement of the joint, and creating a dataset based on the resulting simulation results comprising data relating to the oriental positioning of the joint members for a specific movement and data relating to the points of bone collision provided bone collision is detected by the simulation and wherein in step (iv) the interactive 3D-model is obtained by combining an interactive viewer, the dataset and the 3D-computer model obtained in step (ii).

5. Process according to claim 4, wherein the dataset obtained in step (iii) also comprises data relating to proposed surgical modifications.

6. Process according to any one of claims 1-5, wherein the computer-accessible medium is a PDF document.

7. Process according to any one of claims 1-5, wherein the computer-accessible medium is a HTML document.

8. Process according to any one of claims 1-7, wherein the multimedia object is a device-independent object.

9. Process according to any one of claims 1-8, wherein the image dataset is obtained by a computed tomography (CT) system, a magnetic resonance imaging (MRI) system, a positron emission tomography system, an x-ray device or an ultrasound device.

10. Process according to claim 9, wherein the image dataset is obtained by a computed tomography (CT) system or magnetic resonance imaging (MRI) system.

11. Process according to any one of claims 1-10, wherein the biomechanical or kinematic simulation results relate to the function of deformed joints, of injured joints or joints with planned or already present prosthetic parts.

12. Process according to any one of claims 2-11, wherein the 3D-computer model of the joint is used in step (iii) to analyse a range of motions of the joint, determine situations of bone collisions or situations of no bone collision as the relevant biomechanical or kinematic simulation results and create a multimedia object showing the relevant biomechanical or kinematic simulation results for use in step (iv).

## Patentansprüche

1. Verfahren zum Erzeugen eines Mediums, auf das ein Computer zugreifen kann, das Informationen bezüglich der Funktion eines Gelenks enthält, wobei das Medium, auf das ein Computer zugreifen kann, einen ausführbaren Befehl enthält, um ein Multimedia-Objekt auszuführen durch
(i) Erhalten eine Bild-Datenmenge mit Hilfe einer radiologischen Untersuchung des Gelenks,
(ii) Erstellen eines 3D-Computermodells des Gelenks unter Verwendung der im Schritt (i) erhaltenen Bild-Datenmenge,
(iii) Ausführen eine biomechanischen oder kinematischen Simulation unter Verwendung des Computermodells,
(iv) Empfangen der Ergebnisse der biomechanischen oder kinematischen Simulation als ein Multimedia-Objekt und Integrieren des Multimedia-Objekts in ein Medium, auf das ein Computer zugreifen kann.

2. Verfahren nach Anspruch 1, wobei im Schritt (iii) ein oder mehrere relevante Ergebnisse der biomechanischen oder kinematischen Simulation bestimmt werden und im Schritt (iv) die relevanten Ergebnisse der biomechanischen oder kinematischen Simulation als ein Multimedia-Objekt empfangen und in ein Medium, auf das ein Computer zugreifen kann, integriert werden.

3. Verfahren nach einem der Ansprüche 1-2, wobei das Medium, auf das ein Computer zugreifen kann, ein interaktives 3D-Modell des Gelenks auf der Grundlage des im Schritt (ii) erhaltenen Computermodells enthält und wobei das Medium, auf das ein Computer zugreifen kann, ausführbare Befehle enthält, um mit dem 3D-Modell in Wechselwirkung zu treten.

4. Verfahren nach Anspruch 3, wobei im Schritt (iii) eine biomechanische oder kinematischen Simulation des Gelenks unter Verwendung des 3D-Computermodells ausgeführt wird, wobei mehr als eine Bewegung des Gelenks simuliert wird und wobei eine Knochenkollision bei einer bestimmten Bewegung des Gelenks erfolgen kann, Erzeugen einer Datenmenge anhand der Ergebnisse der resultierenden Simulation, die Daten, die auf die Orientierungspositionierung der Gelenkselemente für eine bestimmte Bewegung bezogen sind, und Daten, die auf die Punkte einer Knochenkollision, sofern eine Knochenkollision durch die Simulation detektiert wird, bezogen sind, enthält, und wobei im Schritt (iv) das interaktive 3D-Modell durch Kombinieren eines interaktiven Betrachters, der Datenmenge und des im Schritt (ii) erhaltenen 3D-Computermodells erhalten wird.

5. Verfahren nach Anspruch 4, wobei die im Schritt (iii) erhaltene Datenmenge außerdem Daten enthält, die auf vorgeschlagene chirurgische Modifikationen bezogen sind.

6. Verfahren nach einem der Ansprüche 1 bis 5, wobei das Medium, auf das ein Computer zugreifen kann, ein PDF-Dokument ist.

7. Verfahren nach einem der Ansprüche 1 bis 5, wobei das Medium, auf das ein Computer zugreifen kann, ein HTML-Dokument ist.

8. Verfahren nach einem der Ansprüche 1 bis 7, wobei das Multimedia-Objekt ein vorrichtungsunabhängiges Objekt ist.

9. Verfahren nach einem der Ansprüche 1 bis 8, wobei die Bild-Datenmenge durch ein Computertomographiesystem (CT-System), ein Magnetresonanz-Bildgebungssystem (MRI-System), ein Positronenemissions-Tomographiesystem, eine Röntgenstrahlvorrichtung oder eine Ultraschallvorrichtung erhalten wird.

10. Verfahren nach Anspruch 9, wobei die Bild-Datenmenge durch ein computertomographisches (CT-System) oder ein Magnetresonanz-Bildgebungssystem (MRI-System) erhalten wird.

11. Verfahren nach einem der Ansprüche 1 bis 10, wobei die Ergebnisse der biomechanischen oder kinematischen Simulation mit der Funktion von verformten Gelenken, von verletzten Gelenken oder von Gelenken mit geplanten oder bereits vorhandenen Prothesenteilen in Beziehung stehen.

12. Verfahren nach einem der Ansprüche 2 bis 11, wobei das 3D-Computermodell des Gelenks im Schritt (iii) verwendet wird, um einen Bereich von Bewegungen des Gelenks zu analysieren, Situationen von Knochenkollisionen oder Situationen ohne Knochenkollision als die relevanten Ergebnisse der biomechanischen oder kinematischen Simulation zu bestimmen und ein Multimedia-Objekt zu erzeugen, das die relevanten Ergebnisse der biomechanischen oder kinematischen Simulation zur Verwendung im Schritt (iv) zeigt.

## Revendications

1. Processus pour générer un support accessible par ordinateur comprenant des informations sur le fonctionnement d'une articulation, dans lequel le support accessible par ordinateur comprend une instruction exécutable pour faire fonctionner un objet multimédia selon les étapes suivantes :
(i) obtenir un ensemble de données d'image à l'aide d'un examen radiologique de l'articulation,
(ii) construire un modèle informatique 3D de l'articulation au moyen de l'ensemble de données d'image obtenu dans l'étape (i),
(iii) exécuter une simulation cinématique ou biomécanique au moyen du modèle informatique,
(iv) recevoir les résultats de simulation cinématique ou biomécanique comme objet multimédia et intégrer l'objet multimédia à un support accessible par ordinateur.

2. Processus selon la revendication 1, dans lequel dans l'étape (iii), un ou plusieurs résultats de simulation cinématique ou biomécanique pertinents sont déterminés et, dans l'étape (iv), les résultats de simulation cinématique ou biomécanique pertinents sont reçus comme objet multimédia et intégrés à un support accessible par ordinateur.

3. Processus selon l'une quelconque des revendications 1 à 2, dans lequel le support accessible par ordinateur comprend un modèle 3D interactif de l'articulation sur la base du modèle informatique tel qu'obtenu dans l'étape (ii) et où le support accessible par ordinateur comprend des instructions exécutables pour interagir avec le modèle 3D.

4. Processus selon la revendication 3, dans lequel, dans l'étape (iii), une simulation cinématique ou biomécanique de l'articulation est exécutée au moyen d'un modèle informatique 3D, où plus d'un mouvement de l'articulation sont simulé et où une collision osseuse peut se produire lors d'un mouvement spécifique de l'articulation, et crée un ensemble de données sur la base des résultats de simulation résultants comprenant des données relatives au position d'orientation des membres de l'articulation pour un mouvement spécifique et des données relatives aux points de collision osseuse, à la condition qu'une collision osseuse soit détectée par la simulation et où dans l'étape (iv), le modèle 3D interactif est obtenu en combinant un visionneur interactif, l'ensemble des données et le modèle informatique 3D obtenu à l'étape (ii).

5. Processus selon la revendication 4, dans lequel l'ensemble de données obtenu à l'étape (iii) comprend également des données relatives à des modifications chirurgicales proposées.

6. Processus selon l'une quelconque des revendications 1 à 5, dans lequel le support accessible par ordinateur est un document PDF.

7. Processus selon l'une quelconque des revendications 1 à 5, dans lequel le support accessible par ordinateur est un document HTML.

8. Processus selon l'une quelconque des revendications 1 à 7, dans lequel l'objet multimédia est un objet indépendant du dispositif.

9. Processus selon l'une quelconque des revendications 1 à 8, dans lequel l'ensemble de données d'image est obtenu par un système de tomodensitométrie (CT), un système d'imagerie par résonnance magnétique (IRM), un système de tomographie par émission de positons, un dispositif de radiographie ou un dispositif à ultrasons.

10. Processus selon la revendication 9, dans lequel l'ensemble de données d'image est obtenu par un système de tomodensitométrie (CT) ou un système d'imagerie par résonnance magnétique (IRM).

11. Processus selon l'une quelconque des revendications 1 à 10, dans lequel les résultats de simulation cinématique ou biomécanique concernent la fonction des articulations déformées, d'articulations blessées ou d'articulations ayant des parties prothétiques déjà existantes ou prévues.

12. Processus selon l'une quelconque des revendications 2 à 11, dans lequel le modèle informatique 3D de l'articulation est utilisé à l'étape (iii) pour analyser une gamme de mouvements de l'articulation, pour déterminer des situations de collision osseuse ou des situations d'absence de collision osseuse comme résultats de simulation cinématique ou biomécanique pertinents et pour créer un objet multimédia montrant les résultats de simulation cinématique ou biomécanique pertinents pour une utilisation à l'étape (iv).
